# EUROPEAN PATENT SPECIFICATION

(11) **EP 0 886 527 B1**
(45) Date of publication and mention of the grant of the patent: **12.09.2001**
(21) Application number: 97905395.6
(22) Date of filing: 27.02.1997
(51) Int. Cl.: A61K 38/21

(54) **PHARMACEUTICAL COMPOSITIONS COMPRISING NATURAL HUMAN ALPHA-INTERFERON**
NATÜRLICHES MENSCHLICHES ALPHA INTERFERON ENTHALTENDE PHARMAZEUTISCHE ZUSAMMENSETZUNGEN
COMPOSITIONS PHARMACEUTIQUES COMPORTANT DE L'INTERFERON ALPHA HUMAIN NATUREL

(30) Priority: 28.02.1996 IT RM960136; 14.06.1996 IT RM960427
(43) Date of publication of application: 30.12.1998
(73) Proprietor: Unihart Corporation, Dublin 2 (IE)
(72) Inventor: TARRO, Giulio IFI Istituto Farmacoterapico, I-00197 Roma (IT); BROZZO, Renzo IFI Istituto Farmacoterapico, I-00197 Roma (IT)
(74) Representative: Bianchetti, Giuseppe, Prof.
(86) International application number: PCT/IT97/00040
(87) International publication number: WO 97/31649

(56) References cited:
- WO-A-88/03411
- ARCHIVUM IMMUNOLOGIAE ET THERAPIAE EXPERIMENTALIS 41 (3-4). 1993. 259-265, XP000674716 GEORGIADES J A: "Early changes in the plasma proteins of patients treated with low doses of oral natural human interferon alpha ( IFN -alpha)."
- ARCHIVUM IMMUNOLOGIAE ET THERAPIAE EXPERIMENTALIS 41 (3-4). 1993. 237-240, XP000674717 RATAJCZAK B: "Observation of the effect of low oral doses of human leukocytic interferon alpha in children with chronic HBV infection and impaired immune responses."
- ARCHIVUM IMMUNOLOGIAE ET THERAPIAE EXPERIMENTALIS 44 (5-6). 1996. 359-366, XP000674715 ZIELINSKA W ET AL: "Comparison of the long-term effects of treatment with oral and parenteral interferon alpha in chronic viral hepatitis patients."

## Description

The invention concerns pharmaceutical compositions for a peroral administration comprising natural human α-interferon isolated from lymphoblastoid or leukocytic cells. In particular the compositions are useful for therapy of viral infections, in particular viral hepatitis, neoplasia and immunodeficiency syndromes. The interferon efficient dosages are clearly lower than dosages utilized for parenteral administration.

α-, β-, γ-interferons are usually administered by injection and are used for therapy. α-interferon is the most largely utilized interferon (1). In an updated study of medicaments for either acute or chronic viral hepatitis therapy (2), only α-interferon is widely accepted as single therapeutic agent.

"Viral hepatitis" means at least five different pathologies, having different agents, namely A, B, C, D, E.

The therapeutic trend is to treat said pathologies with α-interferon, with dosages according to the kind of hepatitis, to the overall status of the subject and to other variable factors. In general, further to the interferon treatment an almost normalisation clinical and biochemical parameters is achieved for chronic hepatitis (B, C, D). The interferon activity on acute hepatitis has not been focused yet, though for hepatitis C, a therapeutic treatment with α-interferon lowers the chronicition rate of the disease.

Therapeutic cycles indicate the daily alternate administration through subcutaneous route of recombinant α-interferon (r α-IFN) at dosages of app. 5.000.000 IU, that in special cases can be up to 9.000.000 IU/day.

The length of therapeutic cycles is of from six months up to one year (nine months average).

In many cases, undesired side effects interfere with the course of therapeutic treatment. In fact some patients, in particular those at an advanced stage of disease or with severe physiologic damages, do not tolerate the therapy and therefore the treatment should be interrupted. Claimed side effects are: fever, nausea, vomit, tiredness, algia and depression.

Moreover the therapeutic cost are quite relevant both due to the high amount of active principle (more than 8.000 new cases each year in Italy and 300.000 world-wide) and to the necessity of hospitalisation just in consideration of said side effects further to the parenteral administration (day hospital or outpatients' department).

Finally, as far as chronic active viral hepatitis is concerned the only alternative to the interferon treatment is represented by liver transplant.

The clinical trend is to increase the posology dosage and the length of therapeutic cycle (3), but clinical data show (4): severe side effects; low acceptance by the patient; high therapeutic costs. Garcia et al. (5) report that the estimate for each cured patient is between 700.000 and 2.000.000 English pounds Capri S. (6) report that the cost of each interferon therapeutic treatment is of Lit. 70.000.000/subject.

It is therefore evident that the actual composition of interferon for therapeutic treatment of hepatitis is not optimal.

Moreover clinical results show a better therapeutic efficacy in patients which are not the main target for therapy, namely: young subjects, subjects with a disease at an initial stage, subjects infected with genotypic virus 2 or 3, low viremia subjects. On the contrary a less therapeutic efficacy can be found in those subjects which really need the therapeutic treatment (subjects low respondent), as subjects affected by an aggressive form (active chronic hepatitis), lonq lenqth diseases affected subjects, over 50 years old subjects. Thus, patients that really need an immediate interferon treatment are those that have a lower chance of success (7).
It is known from Arch. Immunol. Ther. Exp. 41, 259 (1993) to treat patients with chronic hepatitis B virus infection orally with natural human α-interferon-containing lozenges which the patients have to keep on the tongue until complete dissolution. There is, however, a risk of prematurely swallowing them.

Further, from WO-A-88/03411 it is known to administer human α-interferon to animals and humans orally. It is stated that the treatment can be directed, among others, against hyperallergenicity, bacterial infection and viral infection, however, examples of treatment with human α-interferon are reported only with respect to neoplastic disease, autoimmune disorder and acne in humans and for lupus erythematosus, peritonitis, herpesvirus, parvovirus and leukemia in animals.

As dosage formulations there are mentioned lozenges, chewable tablets, mouthwashes (buffered aqueous solutions) and syrups.

There is still a demand for a better oral dosage formulation against hepatitis B and for an oral application at all against other types of hepatitis.

The authors of the instant invention have found a pharmaceutical composition for the treatment of viral hepatitis comprisinq natural human α-interferon from either lymphoblastoid or leukocytic cells to be administered throuqh peroral route, with dosaqes clearly lower than those hitherto used for parenteral and oral administration. The composition maintains unaltered chemical-physical, biological and pharmacological characteristics of the active principle, having a therapeutic effect substantially analogous to the compositions of prior art but overcoming disadvantages thereof.

The composition is administered in a liquid form with a concentration of 100 to 500 I.U./ml, preferably approximately 150 I.U./ml, most preferably in mono-dosage units, most preferably of approximately 1 ml.

The composition acts by activatinq the defence mechanisms against viral hepatitis and stimulates an immune response.

The utilisation of natural interferon was chosen for the better chances of therapeutic success with respect to recombinant interferon, obtained by cloning of a single subtype.

Though leukocytic and lymphoblastoid interferons exert the same therapeutic properties, the former can be advantageously produced. As a matter of fact it is obtainable by stabilised cell lines, without the need of blood donors.

Processes for purifying interferons are known to those skilled in the art, and for example are shown in US Patent 4,732,683; in Cantell K. and Hirvonen S. Texas Reports on Biology and Medicine, Vol. 35, p. 138, 1977; in Zoon K.C. et al. Science 207, p. 527, 1980.

The peroral route is generally much more accepted by subjects, makes easier posology schemes and dosages, lowers or stops the antigenic risk, induces the transmission and amplification signal mechanism, with a mirato therapeutic effect, with dosages 100 times lower than known formulations for parenteral administrations.

The low dosage annuls the risk of toxic effects; allows a better availability of medicine to satisfy an increasing request and a drastic lowering of therapeutic costs.

The preferred formulation in dosage units of small volumes (1 ml) to drink allows an immediate availability of the active principle, a good standard of cleanliness from the monodosage primary container; the certainty of the taken dosage; the taking of the active principle to be immediately absorbed by the oro-pharyngeal mucosa, easily preventing the deglutition, an easy and safe way of administration for all of patients, as opposite to lozenges or tablets formulations that should be kept in the mouth until full dissolution, with high chances of swallowing.

Moreover the composition of the invention is conveniently used for home therapies or on the job place, as precautionary measure for the prophylaxis of viral hepatitis, and to control chronic diseases which need long therapeutic cycles (even yearly) and often recurrent.

The composition can be used also in association with other drugs to get synergism and optimize therapeutic schemes.

The following clinical studies show the therapeutic effect. A comparison of the electrophoretic protein pattern and of the concentration of IgG, IgA, IgM, before the beginning of the peroral therapy with natural human α-interferon of hepatitis affected subjects, before and after two weeks of therapeutic treatment, allows to foresees quali-quantitatively the subject response.

Subjects which respond to the therapy with 450IU/die dosages show a decrease of α2- and β-globulins, of IgGs, of the IgG/IgA ratio, together with an increase of IgA and IgM concentrations, have a good chance of eliminate the HBVe antigen and to seroconvert, namely to confer a stable remission of the pathology.

On the other hand subjects which respond to the same therapy with a decrease of albumin serum concentration, of IgGs, IgAs, IgMs, together to an increase of α1-globulin fractions, should seronvert with longer times.

Moreover subjects that respond with an increase of IgGs, of the IgG/IgA ratio, together with a decrease of IgM and of the IgA/IgM ration, could be resistent to the therapy.

The monitoring of said parameters (markers) is useful for a planning of therapeutic strategies in clinic and also for the clinical practitioner.

### Clinical studies on healthy subjects

Table 1 shows different therapeutic schemes.

**Table 1**

| Experiment | active component | No. admin. /day | Dosages IU per day | day treased | blood bleedings |
|---|---|---|---|---|---|
| A | aA α-IF aB placebo | 1(3dsg) 1(3dsg) | 450 - | 1 1 | T₀, T₁, T₂, T₃, T₀, T₁, T₂, T₃ |
| B | bA α-IF | 1(3dsg) | 450 | 5 | |
| | bB placebo | 1(3dsg) | - | 5 | T₀, T₁, T₂, T₃, T₄, T₅, T₆, T₇ T₀, T₁, T₂, T₃, T₄, T₅, T₆, T₇ |
| C | cA₁ α-IF cA₂ α-IF cb placebo | 2 (1dsg) 3 (1dsg) 3 (1dsg) | 300 450 - | 1 1 1 | T₀, T₁, T₂, T₃ T₀, T₁, T₂, T₃ T₀, T₁, T₂, T₃ |
| D | dA₁ α-IF | 2 (1dsg) | 300 | 5 | |
| | dA₂ α-IF dB placebo | 3 (1dsg) 3 (1dsg) | 450 - | 5 5 | T₀, T₁, T₂, T₃, T₄, T₅, T₆, T₇ T₀, T₁, T₂, T₃, T₄, T₅, T₆, T₇ T₀, T₁, T₂, T₃, T₄, T₅, T₆, T₇ |

T₀ = background; T₁= 1d further the first administration, T₂ = 2d further the first administration, T₃ = 3d further the first administration, T₄ = 4d further the first administration, T₅ = 5d further the first administration, T₆ = 1d after the treatment suspension, T₇ = 2d after the treatment suspension.

The change of the induced biological response with respect to the therapeutic scheme, has been measured on samples of blood, taken at different times. In particular the activity with respect to the day dosage of active principle, to the mono- or pluri-administration, to the length of the therapeutic cycle was measured.

The analysis of data show that natural human α-interferon from either lymphoblastoid or leukocytic cells, administered at low dosages for a peroral route, is able to modulate (according to the dosage and to the length of the therapeutic cycle) the expression of membrane antigen of healthy subject blood mononuclear cells. In particular, according to therapeutic scheme, the pharmaceutical composition seems to be able to increase both CD4 and CD8 cell population. It is also evident an increased expression of markers of cell activation, as DR antigens and interleukin 2 receptor.

The therapeutic scheme with 450IU/die x 5 d (exp. B) is the one providing better results, as shown in Tables 2 and 3. In fact there is an increase (% and absolute) of CD3, CD4, DR1, CD25 lymphocytes. Said increases are, according to different cases, better evident at T₃, T₄, T₅ times to later decrease at T₆ and T₇ times.

The same posology dosage, but with a shorter therapeutic cycle (1 day) (exp.A), interferes less evidently with the % and absolute numbers of mononuclear cells in the blood (Tables 4 and 5). In fact in this experiment an increase of average percentage values but not of absolute T, CD8, and class II hystocompatibility antigen lymphocytes values, is evident at time T₃.

Other experimental conditions show lower increases of the immune response.

Therefore, natural human α-interferon from either lymphoblastoid or leukocytic cells, administered at low dosages trough peroral route, shows an important role in modulating the immune response, both in the phase afferent and efferent, and has a therapeutic application for the treatment of infective diseases and of other conditions of immunodeficiency.

### Clinical studies on hepatitis subjects

### Viral B Hepatitis

14 patients affected by chronic viral B hepatitis, with an age comprised between 4 and 59, were used for random studies.

All of subject were previously treated for different periods ranging from some months to some years with steroids, or with steroid-azothiopurine, with no beneficial effects, neither for the clinical symptomatology nor for the biochemical parameters of the disease, which evolved, in some cases, to hepatic cirrhosis.

The therapeutic treatment of a one administration of 150IU/day was initiated immediately after the suspension of the previous treatment, and effects of said treatment were monitored by checking any alteration of the immune response; of the haematological and biochemical parameters; of serum markers of the viral infection and of the hystochemistry of hepatic bioptic samples.

The time of observation varied from 15 to 32 months and results can be summarized in the following:
1) all of patients during the first 3-6 weeks of treatment registered a transient decay of hepatic biochemical functions (i.e. a 2-3 fold increase of alanineaminetransferase (ALT) levels), with no clinical symptoms of disease worsening;
2) the phenomenon goes on for 4-6 weeks;
3) in all of treated patients an intense activation of the immune system was observed, even after the therapeutic treatment;
4) 7 patients eliminate HBV DNA and HBeAg from serum and stable seroconvert;
5) 1 patient has an HBcAg increased titre, more than the original value;
6) in other 9 patients said -titre decreases significatively.

Therefore, 50% of patients get a stable remission of the disease.

### Viral C Hepatitis

The therapeutic standard of viral hepatitis C foresees the use of α-interferon through parenteral route.

6 active chronic hepatitis C affected patients were subjected to therapy with peroral administration at 150IU/die, by starting the treatment just after the suspension of the steroid therapy.

The observation time (equal to the length of the treatment) resulted to be variable from 19 to 69 weeks. In general the treatment was well tolerated and all of patients registered a significant increase of vivacity and appetite, with a better tolerance to physical exercises.

No patients got a normalization of transaminase levels during the observation period, but one which registered the biochemical and clinical remission of the disease, after the treatment suspension at the 19th week due to an increasing of articular pains.

The results are shown in tables 2-5.

### BIBLIOGRAPHY

1) Howard M. et al. Le Scienze n.311 Vol LIII 72-80 (1994).
2) Saracco G., Rizzetto M. Biomed. Pharmacother. 49 (2), 55-57 (1995).
3) Kasahara A.K., et al., Hepatology; 21, 291-297 (1995).
4) Paoletti A. et al.; Clin. Ter. 146(5), 343-349 (1995).
5) Garcia De Ancos J.L. et al.; J. Hepatol. 11:s11-s18 (1990).
6) Capri S. Adis International, Milano, pp 41-49 (1994).
7) Bianchi F.B., La rivista del medico pratico, Ott. (suppl.5) (1995).

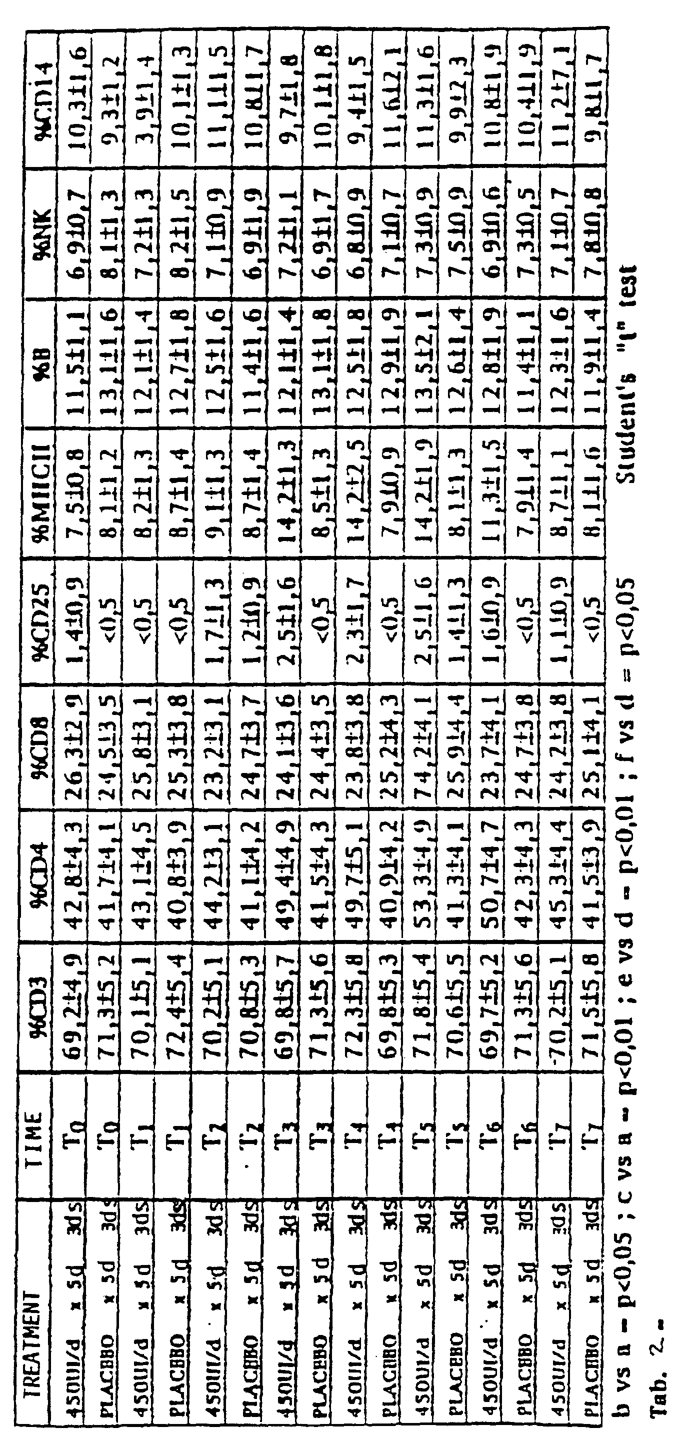

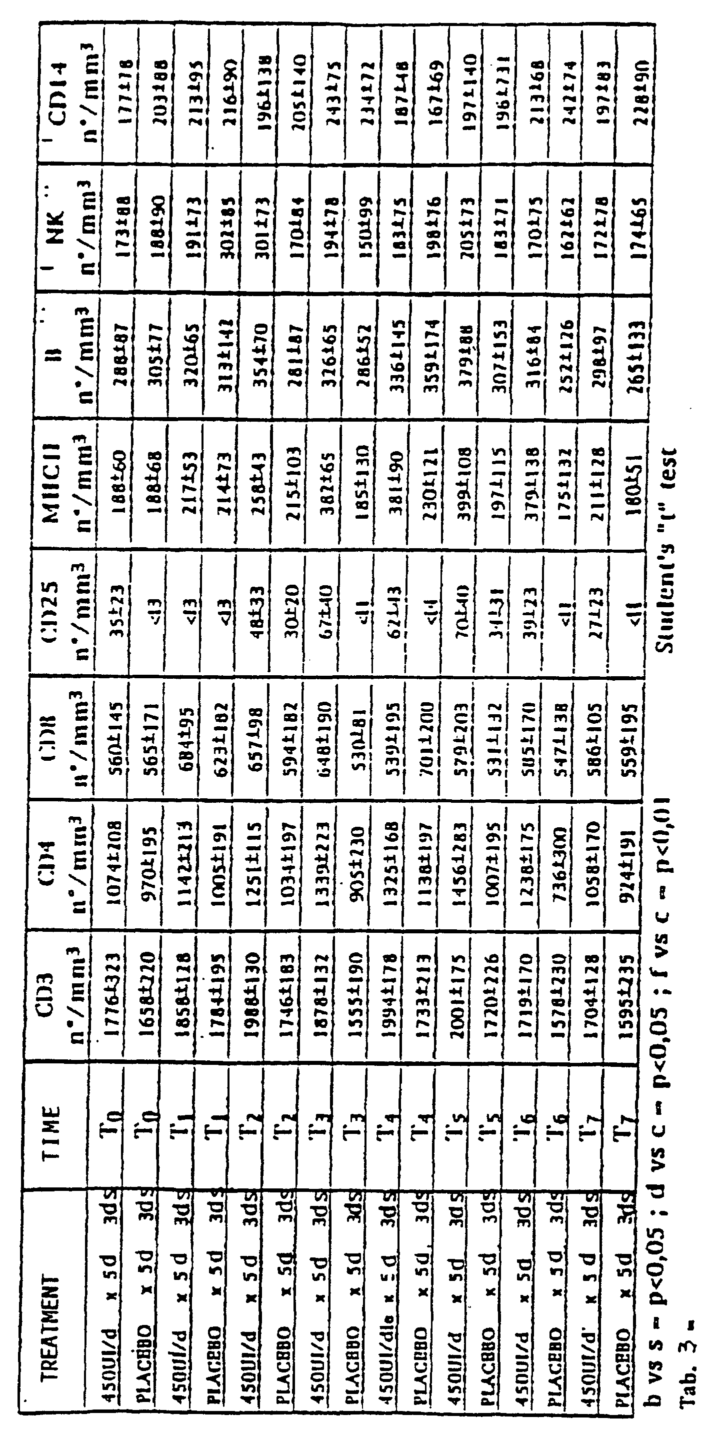

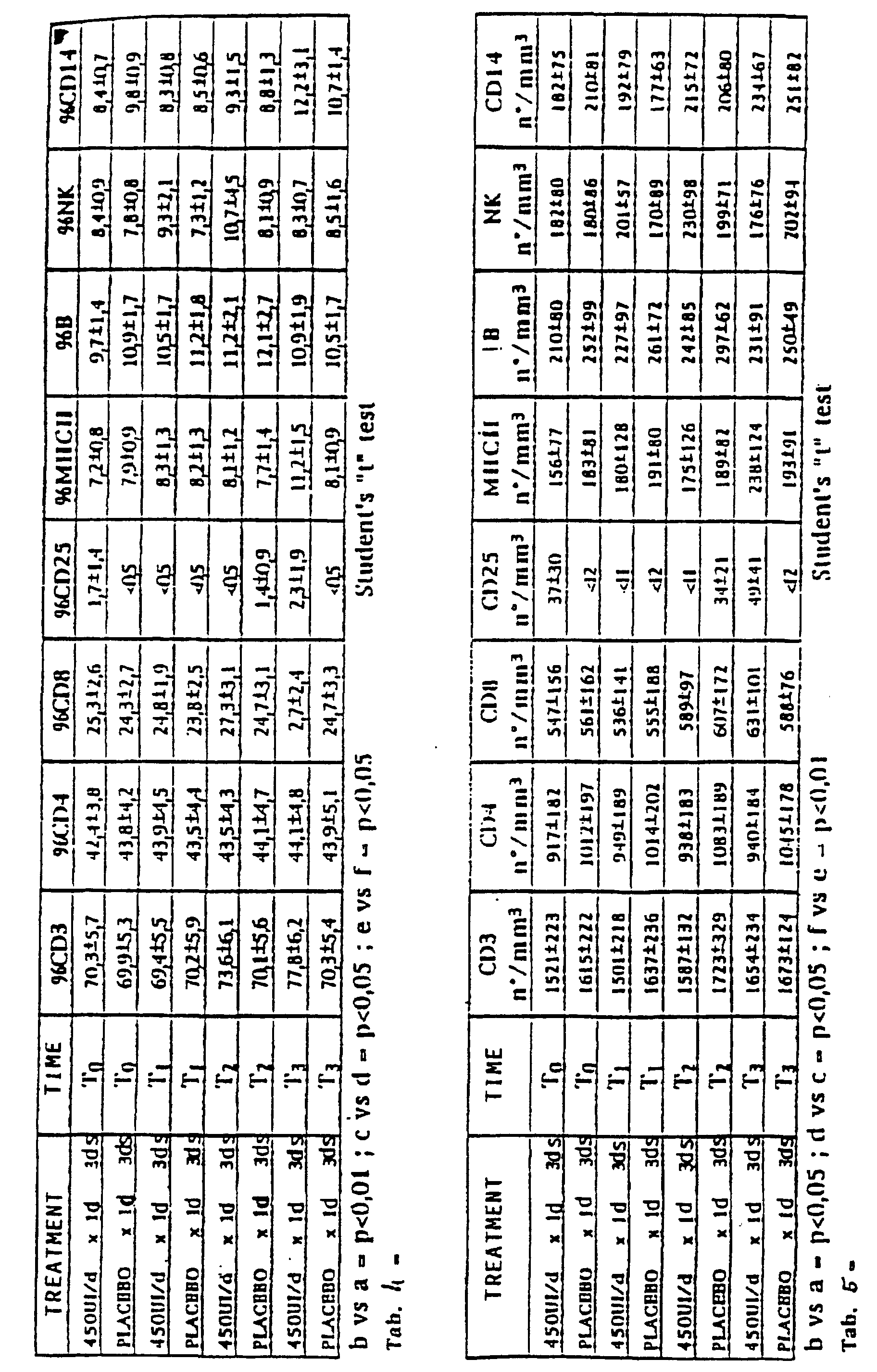

## Claims

1. Use of natural human α-interferon for the preparation of a medicament in liquid form to be administered through peroral route at dosages comprised between 100 IU and 500 IU/day, for therapy of viral hepatitis in humans and animals.

2. Use of natural human α-interferon according to claim 1, wherein said interferon is obtained from lymphoblastoid cell cultures.

3. Use of natural human α-interferon according to claim 1, wherein said interferon is obtained from lymphocyte cells.

4. Use of natural human α-interferon according to any of previous claims wherein said medicament is administered in mono dosage units of approximately 1 ml.

## Patentansprüche

1. Verwendung von natürlichem menschlichen α-Interferon für die Herstellung eines Medikaments in flüssiger Form, das über den peroralen Weg bei Dosen, die zwischen 100 IU und 500 IU/Tag liegen, verabreicht werden soll, für die Therapie von Virushepatitis bei Menschen und Tieren.

2. Verwendung von natürlichem menschlichen α-Interferon nach Anspruch 1, worin das Interferon aus Lymphoblastoidzellkulturen erhalten wird.

3. Verwendung von natürlichem menschlichen α-Interferon nach Anspruch 1, worin das Interferon aus Lymphozytenzellen erhalten wird.

4. Verwendung von natürlichem menschlichen α-Interferon nach einem der vorhergehenden Ansprüche, worin das Medikament in Einzeldosiseinheiten von etwa 1 ml verabreicht wird.

## Revendications

1. Utilisation de l'α-interféron naturel humain pour la préparation d'un médicament sous forme liquide à administrer par voie buccale à des doses comprises entre 100 et 500 UI/jour, pour la thérapie des hépatites virales chez les humains et les animaux.

2. Utilisation de l'α-interféron naturel humain selon la revendication 1, dans laquelle ledit interféron est obtenu à partir de cultures de cellules lymphoblastoides.

3. Utilisation de l'α-interféron naturel humain selon la revendication 1, dans laquelle ledit interféron est obtenu à partir de cellules lymphocytes.

4. Utilisation de l'α-interféron naturel humain selon l'une quelconque des revendications précédentes, dans laquelle ledit interféron est administré en doses unitaires d'environ 1 ml.
